# EUROPEAN PATENT APPLICATION

(11) **EP 1 612 543 A1**
(43) Date of publication of application: **04.01.2006**
(21) Application number: 04725997.3
(22) Date of filing: 06.04.2004
(51) Int. Cl.: G01N 21/78, B01D 46/42, F24F 1/00, F24F 1/02, F24F 5/00

(54) **COLORING SENSOR**

(30) Priority: 07.04.2003 JP 2003102683; 10.10.2003 JP 2003352417
(71) Applicant: DAIKIN INDUSTRIES, LIMITED, kita-ku, 530-8323 Osaka (JP)
(72) Inventor: AMANO, Yoshihisa, Tsukuba-shi, Ibaraki 3050841 (JP); ARAI, Jun-ichiro, Tsukuba-shi, Ibaraki 3050841 (JP); KATAYAMA, Hideo, Tsukuba-shi, Ibaraki 3050841 (JP)
(74) Representative: Goddar, Heinz J.
(86) International application number: PCT/JP2004/004941
(87) International publication number: WO 2005/012905

(57) **Abstract**

A means for detecting a presence of a particular material in the air, a filter for an air conditioner which can confirm the presence of the particular material in the air, and a method for confirming a performance of the filter for an air conditioner about a removal of the particular material and/or a lifetime of the filter for an air conditioner are provided.

The particular material present in the air is detected by a color change of a chromogenic sensor comprising a receptor molecule specifically binding with the particular material in the air, and a polymer molecule whose light absorbency is altered due to binding of the particular material and the receptor molecule.

## Description

### FIELD OF THE INVENTION

The present invention relates to a colorimetric sensor. More particularly, it relates to a colorimetric sensor for detecting a particular material present in the air. Moreover, it relates to a filter or apparatus for an air conditioner equipped with the colorimetric sensor, and to an air conditioner equipped with such the filter or apparatus.

### BACKGROUND OF THE INVENTION

As a means for confirming a lifetime of a filter for an air conditioner, a lifetime display which utilizes a dust-collecting function of a nonwoven fabric has been known. This has a system that the filter is covered with a colored nonwoven fabric consisting of fibers excellent in the dust-collecting function and an apparent color of the nonwoven fabric is changed depending upon a quantity of a dust adsorbed onto the nonwoven fabric with the passage of the air therethrough (For example, see JP-B 32860/1995).

On the other hand, different from the dust, a presence of a material in the air such as bacteria, viruses, VOCs (volatile organic compounds) etc. is difficult to be visually confirmed even when it is adsorbed onto the filter. As a method for detecting such the material, a measurement has been known, in which a physiologically active material and an environmental pollutant etc. such as natural materials, toxins, hormones, agrochemicals etc. are captured by an antigen-antibody reaction with an antibody (or an antigen) specific for the material, and the material captured by the antibody is measured according to an immunological analytical method etc, with a labeled secondary antibody etc (For example, see JP-A 202307/2002).

### SUMMARY OF THE INVENTION

While various filters have been developed in order for a removal of a particular material present in the air such as, for example, bacteria, viruses, VOCs (volatile organic compounds) etc., there has been no means which not only reveals a presence of such the particular material, but also confirms a performance of the filter about the removal of the material or a lifetime of the filter. That is, since to what an extent the particular material in the air has been removed by the filter and to what an extent the particular material in the air has been adsorbed onto the filter could not be known, there were difficulties in confirming a working state of the filter and in exchanging the filter at a suitable time to use under an optimal condition.

Therefore; the present invention provides a novel solving means for detecting the presence of the particular material in the air. Moreover, the present invention provides a filter for an air conditioner which can confirm the presence of the particular material in the air, and a method for confirming the performance of the filter for an air conditioner about the removal of the particular material and/or the lifetime of the filter.

The present invention is based on a novel concept that a colorimetric sensor, which comprises a receptor molecule specifically binding with a particular material in the air and a polymer molecule whose light absorbency is altered due to binding of the particular material and the receptor molecule, is used as a means for detecting the presence of the particular material in the air.

That is, the present invention provides:
(1) a colorimetric sensor for detecting a particular material in the air, comprising a receptor molecule specifically binding with the particular material in the air, and a polymer molecule whose light absorbency is altered due to binding of the particular material and the receptor molecule;

(2) the colorimetric sensor according to (1), wherein said receptor molecule is linked to the polymer molecule at a portion of the receptor molecule not participating in binding with the particular material;

(3) the colorimetric sensor according to (1) or (2), wherein said alteration in light absorbency of the polymer molecule is caused by a molecular structural alteration the polymer molecule;

(4) the colorimetric sensor according to (3), wherein said polymer molecule is polydiacetylene;

(5) the colorimetric sensor according to (1) or (2), wherein said alteration in light absorbency of the polymer molecule is caused by an alteration in an electron distribution state in the polymer molecule;

(6) the colorimetric sensor according to (5), further comprising a complex consisting of an electron-withdrawing material and a ligand specific for the receptor molecule, wherein said complex is linked to the receptor molecule via the ligand;

(7) the colorimetric sensor according to (5) or (6), wherein said polymer molecule is selected from a group consisting of polythiophene, oligothiophene, polypyrrole and polyvinylcarbazole;

(8) the colorimetric sensor according to (7), wherein said polymer molecule is polyvinylcarbazole;

(9) the colorimetric sensor according to any one of (6) to (8), wherein said ligand is selected from a group consisting of viruses, antigens and biotin;

(10) the colorimetric sensor according to any one of (6) to (9), wherein said electron-withdrawing material is selected from a group consisting of anthraquinone, tetracyanoquinodimethane, trinitrofluorenone and dinitrofluorenone;

(11) the colorimetric sensor according to any one of (1) to (10), wherein said receptor molecule is selected from a group consisting of sialic acid, ganglioside, antibodies, antibody fragments and avidin;

(12) the colorimetric sensor according to any one of (1) to (11), further comprising a water-retaining means;

(13) the colorimetric sensor according to (12), wherein said water-retaining means is a porous material;

(14) the colorimetric sensor according to (13), wherein said porous material is selected from a group consisting of zeolite and porous sintered products;

(15) the colorimetric sensor according to (12), wherein said water-retaining means is an absorbent polymer;

(16) the colorimetric sensor according to (15), wherein said absorbent polymer is selected from a group consisting of alginic acid, dextran, collagen, cellulose derivatives, starch derivatives, polyvinyl alcohol and sodium polyacrylate;

(17) the colorimetric sensor according to (16), wherein said cellulose derivative is selected from a group consisting of carboxymethylcellulose, methylcellulose and ethylcellulose;

(18) the colorimetric sensor according to any one of (1) to (11), wherein said polymer molecule is modified so as to have a water-absorbing ability;

(19) a filter for an air conditioner equipped with the colorimetric sensor as defined in any one of (1) to (18);

(20) an apparatus for confirming a lifetime of a filter for an air conditioner, comprising a solution containing the colorimetric sensor as defined in any one of (1) to (18), a solution bath for retaining the solution, and a means for bubbling the air before and/or after passing through the filter in the solution;

(21) an air conditioner equipped with the filter as defined in (19);

(22) an air conditioner equipped with the apparatus as defined in (20);

(23) the air conditioner according to (21) or (22), wherein said colorimetric sensor is placed at an upstream and/or downstream side of the filter and is placed so as to contact with the air which has not been heat-exchanged;

(24) the air conditioner according to any one of (21) to (23), wherein said colorimetric sensor is controlled so as to be maintained at a suitable temperature for binding with the particular material without depending upon a working state of the air conditioner;

(25) the air conditioner according to any one of (21) to (24), further comprising an optical sensor for detecting a color change of the colorimetric sensor; and

(26) a method for confirming a lifetime of a filter for an air conditioner, comprising using the colorimetric sensor as defined in any one of (1) to (18).

According to the colorimetric sensor of the present invention, the presence of the particular material in the air can be confirmed by the color change of the sensor, and the working state of the filter about the removal of the particular material can be monitored. Moreover, according to the colorimetric sensor of the present invention, a suitable time for exchanging the filter can be known, and an effective state for using the filter can be established. Furthermore, when the color change of the colorimetric sensor has become to be not caused any more due to binding saturation of the particular material in the air, the colorimetric sensor of the present invention can be reused by treating with a suitable reagent.

### DETAILED DESCRIPTION OF THE PREFERRED EMBODIMENTS

The colorimetric sensor of the present invention comprises a receptor molecule specifically binding with a particular material in the air, and a polymer molecule whose light absorbency is altered due to binding of the receptor molecule with the particular material. In the colorimetric sensor of the present invention, the receptor molecule is linked to the polymer molecule at a portion of the receptor molecule not participating in binding with the particular material.

Examples of the "particular material" of the present invention are not limited to, but include, for example, bacteria, viruses, antigens, VOCs (volatile organic compounds), toxins and biotin etc.

The receptor molecule of the present invention is not particularly limited so long as it has a capability of specifically binding with the particular material in the air. Examples of the receptor molecules for the particular material of the present invention include, for example, sialic acid when the particular material is influenza virus, ganglioside when the particular material is a cholera toxin, an intact antibody such as of IgG etc. or an antibody fragment such as F(ab')2, Fab, etc., binding to a particular antigenic determinant of bacteria when the particular material is bacteria, and avidin etc. when the particular material is biotin.

In the first aspect of the present invention, light absorbency of the polymer molecule of the present invention is altered due to a structural alteration therein by application of a physical force from outside. In this aspect, the receptor molecule of the present invention is incorporated into the polymer molecule to impart, in advance, the structural alteration to the polymer molecule to such an extent that a color change thereof is not caused. When the particular material binds to the receptor molecule, the physical force is applied to a neighboring polymer molecule due to the structural alteration in the receptor molecule and, thereby, light absorbency of the polymer molecule is altered, and a color of the chromogenic sensor is changed. In this aspect, suitable examples of the polymer molecule include, for example, polydiacetylene (PDA) etc. Examples of a shape of the polymer molecule include, for example, a built-up film (LB film) in which monomer units are regularly orientated, a liposome or a film etc. formed by polymerization of the LB film. A solid of the polymer molecule may be also used.

In the second aspect of the present invention, light absorbency of the polymer molecule of the present invention is altered due to an alteration in an electron distribution state in the molecule. In this aspect, the electron-withdrawing material is bonded to the receptor molecule of the present invention in advance. The electron-withdrawing material is bonded to the receptor molecule via a suitable ligand specific for the receptor molecule. At this time, an electron in the polymer molecule linked to the receptor molecule has been drawn to a side of the electron-withdrawing material due to an interaction with the electron-withdrawing material. Simultaneously with binding of the particular material in the air to the receptor molecule, the electron-withdrawing material, which has been bonded to the receptor molecule, is desorbed from the receptor molecule and the electron in the polymer molecule transfers to cause the alteration in the electron distribution state, and consequently, light absorbency of the polymer molecule is altered. In this aspect, examples of a suitable polymer molecule include, for example, polythiophene, oligothiophene, polypyrrole and polyvinylcarbazole etc. Among them, polyvinylcarbazole is particularly preferable. Examples of a shape of the polymer molecule include, for example, a built-up film (LB film) in which monomer units are regularly orientated, a liposome or film formed with polymerization of the LB film, and fibers of the polymer molecule etc. A solid of the polymer molecule may be also used. Examples of the "electron-withdrawing material" include, for example, a compound having an electron-withdrawing group such as a cyano group and a nitro group etc. Examples of a suitable electron-withdrawing material include, for example, anthraquinone, tetracyanoquinodimethane, trinitrofluorenone, dinitrofluorenone etc. Examples of the "ligand specific for a receptor molecule" include those defined as the aforementioned "particular material", but an optional naturally-occurring or synthesized chemical material may be used so long as it specifically binds to the receptor molecule of the present invention.

A complex of the electron-withdrawing material and the ligand can be prepared by covalently binding both of them. Those skilled in the art can freely select a suitable combination of the ligand and the electron-withdrawing material depending upon a kind of the receptor molecule and the polymer molecule to be used, can properly select a reaction for covalently binding both of them and can perform the reaction. The resulting complex can be bonded to the receptor molecule, for example, by contacting with the receptor molecule in a solution.

In this aspect, since the color of the colorimetric sensor of the present invention is changed due to a reversible alteration in the electron distribution state in the polymer molecule, the colorimetric sensor of the present invention can be reused by restoring an initial electron distribution state in the polymer molecule even when the color change of the colorimetric sensor has become to be not caused any more due to binding saturation of the particular material. For example, the colorimetric sensor can be restored by immersing in a solution containing the complex of the electron-withdrawing material and the ligand to substitute the particular material bonded to the receptor molecule with the complex.

The colorimetric sensor of the present invention may be produced in various shapes. For example, when it is produced as a liposome-type colorimetric sensor, first, an LB film of the monomer molecule may be prepared in a suitable organic solvent. Then, the colorimetric sensor of the present invention may be produced by stirring the resulting LB film and a predetermined amount of the receptor molecule in a suitable buffer and sonicating it, thereafter, polymerizing the monomer molecule by UV irradiation. When the colorimetric sensor is produced as a film-like colorimetric sensor, it may be produced by transferring the LB film of the monomer molecule, which is formed with a LB film built-up method according to conventional procedures, to a suitable holder, immersing it in a solution containing a predetermined amount of the receptor molecule and, thereafter, polymerizing the monomer molecule by UV irradiation. Those skilled in the art can easily produce the colorimetric sensor of the present invention according to a procedure, for example, as described in JP-A 194130/1999 or Song et al., *Biomedical Microdevices,* Vol. 4, No.3, pp.211-219, 2002. Moreover, when it is produced as a fibrous colorimetric sensor, it may be produced by dissolving a monomer of the polymer molecule and a predetermined amount of the receptor molecule in a suitable solution, injecting the solution through a nozzle and irradiating UV thereto. Alternatively, the polymer molecule of the present invention may be prepared by a synthesis such as radical polymerization or cationic polymerization etc. of the monomer molecule.

In the present invention, plural kinds of colorimetric sensors for detecting different particular materials may be prepared with respective receptor molecules, specific for various particular materials, and they may be used simultaneously. In this way, the presence of various particular materials may be individually and simultaneously confirmed, and the performance of the filter about the removal of an individual particular material may be confirmed, by using plural kinds of colorimetric sensors for detecting the particular material.

The filter used in the present invention is not particularly limited so long as it can collect the particular material in the air. Examples of a material constituting the filter include, for example, a woven or nonwoven fabric made of fibers such as acrylic fibers, cellulose fibers, glass fibers, polystyrene fibers, polyethylene fibers, polypropylene fibers etc. ceramics, and organic polymer films processed into porous by foaming etc. Examples of a preferable filter of the present invention include, for example, a filter which is made of cellulose fibers carrying sialic acid and can capture influenza virus, as described in JP-A 527166/2001. Alternatively, the colorimetric sensor of the present invention formed in a fibrous shape may be used as a material for processing into the filter.

The filter of the present invention may be used by directly attaching to an air conditioner. Alternatively, the filter may be used as a constituent of one member of a filter unit for an air conditioner. Such the filter unit which is produced with the filter as a constituent of one member is also within a scope of the present invention.

Examples of a shape of the filter of the present invention include a honeycomb structure, and a shape formed by processing into a reticular shape, a cloth-like shape, or a pellet-like shape and sealing this (for example, placing this in a bag, which is used as a filter as it is) etc.

In one aspect of the present invention, the colorimetric sensor of the present invention may be used with retaining on the filter. Examples of a method for retaining the colorimetric sensor on the filter include, for example, a physical adsorption onto a filter fiber, a chemical modification and a use of a binder etc. Preferably, the colorimetric sensor of the present invention is retained on a porous material, and the porous material is further retained on the filter.

In a preferable aspect, the filter of the present invention comprises a water-retaining means for retaining and supplying a moisture necessary for binding of the colorimetric sensor and the particular material in the air. Examples of the water-retaining means include, for example, a porous material and an absorbent polymer etc. Moreover, a polymer to which a water-absorbing ability is imparted by modifying with a suitable group may be also used. The porous material is preferable, since not only it can immobilize the colorimetric sensor of the present invention on the filter, but also it can supply water which serves as a medium for a binding reaction of the receptor molecule and the particular material, by adsorbing a moisture in the air. Moreover, the porous material may have an effect of selecting and collecting the particular materials present in the air depending upon its pore diameter. Examples of the porous material include, for example, zeolite, clay, porous silica and diatomaceous earth etc. Example of the absorbent polymer include, for example, alginic acid, dextran, collagen, cellulose derivatives, starch derivatives, polyvinyl alcohol and sodium polyacrylate etc.

In another aspect of the present invention, the colorimetric sensor of the present invention may be used without retaining on the filter. Specifically, for example, the colorimetric sensor of the present invention may be used in a solution. In this case, the air may be contacted with the colorimetric sensor in a solution containing the colorimetric sensor to bind the particular material in the air with the colorimetric sensor by bubbling the air through the solution, and the presence of the particular material can be confirmed by the color change of the solution.

A solvent to be used for the solution containing the colorimetric sensor is not particularly limited so long as it can dissolve the colorimetric sensor of the present invention and it serves as a medium for the binding reaction of the receptor molecufe of the colorimetric sensor and the particular material. Examples of the solvent include water, and a non-aqueous solvent such as ethanol, isopropanol, hydrocarbon etc. Moreover, other ingredient contributing to the binding reaction of the receptor molecule and the particular material may be further added into the solution. Examples of such the ingredient include a buffering agent for adjusting the solution to and maintaining at a suitable pH for the binding reaction (for example, Tris, EDTA, phosphates etc.) etc.

A means for bubbling the air through the solution is for contacting the colorimetric sensor of the present invention with the air in the solution, and it is constructed of a pump, a gas-feeding tube, a nozzle etc. for feeding and releasing to the solution the air which has been collected by a gas-collecting vessel etc. The bubbling promotes the binding reaction of the receptor molecule of the colorimetric sensor and the particular material by a contact of the colorimetric sensor in the solution and the air with a stirring effect of the solution generation of the bubble.

In another aspect of the present invention, there is provided an apparatus which comprises a solution containing the colorimetric sensor of the present invention, a solution bath for placing the solution therein, and the aforementioned means for bubbling. Using this apparatus, the air processed or to be processed by the filter of the present invention is bubbled through the solution containing the colorimetric sensor of the present invention to contact the colorimetric sensor and the air. The presence of the particular material can be confirmed by the color change of the colorimetric sensor in the solution and, for example, it can be confirmed that the lifetime of the filter is completed when a color thereof is fully changed.

The colorimetric sensor of the present invention may be used by placing it at an upstream and/or downstream side of the filter whether the colorimetric sensor is used with or without retaining on the filter.

In the case where the colorimetric sensor is placed at the upstream side of the filter, that is, it is placed so as to contact with the air before passing through the filter, the color change of the colorimetric sensor reflects an accumulative amount of the particular material present in the air before passing through the filter. For example, if the color change of the colorimetric sensor reflecting an acceptable amount of the particular material on the filter is determined in advance, for example, by an experiment, the lifetime of the filter can be confirmed at the time the color of the colorimetric sensor is fully changed to a determined color change. Moreover, to what an extent an environment has been polluted by the particular material can be also confirmed by monitoring the color change of the colorimetric sensor with time. For example, in the case where the color of the colorimetric sensor is suddenly changed, it is expected that the environment is highly polluted by the particular material.

In the case where the colorimetric sensor is placed at the downstream side of the filter, that is, it is placed so as to contact with the air after passing through the filter, the color change of the colorimetric sensor reflects an accumulative amount of the particular material remaining in the air after passing through the filter. Therefore, if the color change of the colorimetric sensor is monitored with time, whether the performance of the filter about the removal of the particular material is maintained can be confirmed. That is, in the case where the color of the colorimetric sensor is suddenly changed, it is expected that the performance of the filter about the removal is lowered or an amount of an adsorbed particular material is beyond an acceptable amount of the filter.

Moreover, in the case where the colorimetric sensors are placed at both of the upstream and downstream sides of the filter, to what an extent the particular material in the air has been removed by the filter can be confirmed. Moreover, a change in the performance of the filter about the removal with time can be monitored by monitoring a difference in the color change of the colorimetric sensors between at the upstream and downstream sides with time at particular intervals. That is, it is shown that the performance of the filter about the removal is decreasing as the difference in the color change of the colorimetric sensors between at the upstream and downstream sides at particular intervals is decreasing. When there is no difference, it is shown that the performance of the filter about the removal is lost and the lifetime of the filter is completed.

The color change of the colorimetric sensor can be confirmed by directly observing the colorimetric sensor visually. Alternatively, a display window etc. for conveniently observing the color change of the colorimetric sensor may be provided, and one can visually observe the color change of the colorimetric sensor through the display window.

In another aspect of the present invention, the color change of the colorimetric sensor of the present invention can be confirmed by another detecting means. Examples of such the detecting means include, for example, an optical sensor. It is possible that a minute color change of the colorimetric sensor which can not be visually confirmed is easily detected by detecting the color change of the colorimetric sensor with the optical sensor and converting it into an electric signal. Furthermore, such the electric signal may be processed into various information about the presence of the particular material or the working state of the filter etc. and they may be displayed.

Moreover, the present invention also provides an air conditioner equipped with the colorimetric sensor of the present invention retained or not retained on the filter. The term "air conditioner" inclusively refers to an apparatus for maintaining a room environment, for example, temperature, humidity, airflow, a presence of bacteria, dusts, odors and harmful materials etc., at an optimal condition for an organism (including a human) or a product in the room. In addition to common air conditioners, it includes air purifiers and ventilators etc.

The colorimetric sensor of the present invention may be deteriorated at a higher temperature due to degeneration etc., and an adequate reaction with the particular material may not be caused in the colorimetric sensor at a lower temperature in some cases. Preferably, the air conditioner of the present invention is designed such that the colorimetric sensor is contacted with the air which has not been heat-exchanged. More preferably, the air conditioner of the present invention is designed such that the colorimetric sensor is maintained at a suitable temperature for binding with the particular material, without depending upon a working state thereof. For example, the air conditioner of the present invention is provided with an isolation mechanism for maintaining the colorimetric sensor at a lower temperature by utilizing a chill of a heat exchanger during an air cooling-driving, and for shutting off airflow from the heat exchanger during an air heating-driving.

The present invention will be further illustrated in more detail by way of Examples, but the present invention is not limited thereto.

### EXAMPLE

Example 1

### Liposome-type colorimetric sensor for detecting influenza virus

Sialic acid was chemically modified to form S-glycoside according to a procedure described in Song et al., *Biomedical Microdevices* Vol.4, No.3, pp.211-219, 2002. An S-linked diacetylene ligand of sialic acid was prepared which can link to PDA via an S-glycoside linkage. The S-linked diacetylene ligand in an amount of 5 mole % was mixed with a PDA matrix in a 0.1N NaCl aqueous solution (0.15 mg/mL). The resulting suspension was sonicated for 20 minutes, and incubated at 4 degrees Celsius for 90 minutes. The resulting liposome solution was transferred to a 96-well plate (100 µL/well) and polymerized with UV irradiation at 254 nm to obtain a PDA liposome into which sialic acid was incorporated.

Example 2

### Preparation of filter with colorimetric sensor for influenza virus

The liposome-type colorimetric sensor obtained in aforementioned Example 1 was dissolved in a 0.1 mM NaCl aqueous solution and the solution was stirred. Then, zeolite (molecular sieve 13x (Product Number 25960-08), Kanto Chemical Co., Inc.) was immersed in the solution to adsorb the colorimetric sensor onto zeolite. A cellulose fiber to which a sialic acid residue was linked via isocyanate was prepared, and a filter which can capture influenza virus was prepared with the fiber, according to a method described in JP-A 52716612001. Separately, a suspension was prepared by suspending in water zeolite with the colorimetric sensor adsorbed thereonto as described above, and a binder (Snowtex O-40, Nissan Chemical Industries, LTD.) in half an amount (v/v) of the suspension was added thereto and the mixture was stirred. Then, the resulting filter as described above was immersed in the suspension, and zeolite was adsorbed and fixed on the filter with gently stirring and, thereafter, the filter was removed.

Example 3

### Detection of influenza virus with colorimetric sensor of the present invention

The filter equipped with the colorimetric sensor obtained in Example 2 is placed on an air conditioner. The air conditioner is driven to successively feed the air containing influenza virus to the filter. A color of the colorimetric sensor retained on the filter turns from blue to red, and thereby, the presence of influenza virus in the air can be visually confirmed.

Example 4

### Film-type colorimetric sensor for detecting Japanese cedar pollen

### (1) Synthesis of polymer molecule

### (a) Synthesis by radical polymerization

A benzene solution containing an *N*-vinylcarbazole monomer (3.7×10⁻¹ mol dm⁻³) and an initiator (2,2'-azobis(isobutyronitrile)(AIBN), 3.7x10⁻³ mol dm⁻³) was freeze-degassed, and then stirred at 70 degrees Celsius for 48 hours. Thereafter, the reaction solution was poured into a methanol solution to obtain a yellow precipitate. A reprecipitation of the crude product was performed three times with tetrahydrofuran/n-hexane to obtain a desired polymer.

(b) Synthesis by cationic polymerization
A dichloromethane solution containing an *N*-vinylcarbazole monomer (5.0×10⁻² mol dm⁻³) and an initiator (boron trifluoride ethyl etherate)( BF₃ · Et₂O) 9.0×10⁻⁴ mol dm⁻³) was freeze-degassed, and then stirred at 0 degree Celsius for 8 hours. Thereafter, the reaction solution was poured into a methanol solution to obtain a yellow precipitate. A reprecipitation of the crude product was performed three times with benzene/methanol to obtain a desired polymer.

(2) Production of colorimetric sensor
An antibody against Japanese cedar pollens is mixed with the aforementioned polymer molecule, and the mixture is dissolved in an organic solvent such as benzene, tetrahydrofuran and chloroform etc. to form a film according to a rotational application method or a casting method. Separately, a Japanese cedar pollen antigen is bonded with dinitrobenzoic acid in a solution to form a complex. The complex is reacted with the resulting film to obtain a desired colorimetric sensor for the Japanese cedar pollen.

## Claims

1. A colorimetric sensor for detecting a particular material in the air, comprising a receptor molecule specifically binding with the particular material in the air, and a polymer molecule whose light absorbency is altered due to binding of the particular material and the receptor molecule.

2. The colorimetric sensor according to claim 1, wherein said receptor molecule is linked to the polymer molecule at a portion of the receptor molecule not participating in binding with the particular material.

3. The colorimetric sensor according to claim 1 or 2, wherein said alteration in light absorbency of the polymer molecule is caused by a molecular structural alterationin the polymer molecule.

4. The colorimetric sensor according to claim 3, wherein said polymer molecule is polydiacetylene.

5. The colorimetric sensor according to claim 1 or 2, wherein said alteration in light absorbency of the polymer molecule is caused by an alteration in an electron distribution state in the polymer molecule.

6. The colorimetric sensor according to claim 5, further comprising a complex consisting of an electron-withdrawing material and a ligand specific for the receptor molecule, wherein said complex is linked to the receptor molecule via the ligand.

7. The colorimetric sensor according to claim 5 or 6, wherein said polymer molecule is selected from a group consisting of polythiophene, oligothiophene, polypyrrole and polyvinylcarbazole.

8. The colorimetric sensor according to claim 7, wherein said polymer molecule is polyvinylcarbazole.

9. The colorimetric sensor according to any one of claims 6 to 8, wherein said ligand is selected from a group consisting of viruses, antigens and biotin.

10. The colorimetric sensor according to any one of claims 6 to 9, wherein said electron-withdrawing material is selected from a group consisting of anthraquinone, tetracyanoquinodimethane, trinitrofluorenone and dinitrofluorenone.

11. The colorimetric sensor according to any one of claims 1 to 10, wherein said receptor molecule is selected from a group consisting of sialic acid, ganglioside, antibodies, antibody fragments and avidin.

12. The colorimetric sensor according to any one of claims 1 to 11, further comprising a water-retaining means.

13. The colorimetric sensor according to claim 12, wherein said water-retaining means is a porous material.

14. The colorimetric sensor according to claim 13, wherein said porous material is selected from a group consisting of zeolite and porous sintered products.

15. The colorimetric sensor according to claim 12, wherein said water-retaining means is an absorbent polymer.

16. The colorimetric sensor according to claim 15, wherein said absorbent polymer is selected from a group consisting of alginic acid, dextran, collagen, cellulose derivatives, starch derivatives, polyvinyl alcohol and sodium polyacrylate.

17. The colorimetric sensor according to claim 16, wherein said cellulose derivative is selected from a group consisting of carboxymethylcellulose, methylcellulose and ethylcellulose.

18. The colorimetric sensor according to any one of claims 1 to 11, wherein said polymer molecule is modified so as to have a water-absorbing ability.

19. A filter for an air conditioner equipped with the colorimetric sensor as defined in any one of claims 1 to 18.

20. An apparatus for confirming a lifetime of a filter for an air conditioner, comprising a solution containing the colorimetric sensor as defined in any one of claims 1 to 19, a solution bath for retaining the solution, and a means for bubbling the air before and/or after passing through the filter in the solution.

21. An air conditioner equipped with the filter as defined in claim 19.

22. An air conditioner equipped with the apparatus as defined in claim 20.

23. The air conditioner according to claim 21 or 22, wherein said colorimetric sensor is placed at an upstream and/or downstream side of the filter and is placed so as to contact with the air which has not been heat-exchanged.

24. The air conditioner according to any one of claims 21 to 23, wherein said colorimetric sensor is controlled so as to be maintained at a suitable temperature for binding with the particular material without depending upon a working state of the air conditioner.

25. The air conditioner according to any one of claims 21 to 24, further comprising an optical sensor for detecting a color change of the colorimetric sensor.

26. A method for confirming a lifetime of a filter for an air conditioner, comprising using the colorimetric sensor as defined in any one of claims 1 to 18.
